# EUROPEAN PATENT SPECIFICATION

(11) **EP 2 662 039 B1**
(45) Date of publication and mention of the grant of the patent: **06.03.2019**
(21) Application number: 13167146.3
(22) Date of filing: 08.05.2013
(51) Int. Cl.: A61B 18/00, A61M 25/01, A61B 17/00, A61M 39/10, A61B 18/18, A61B 18/14, A61M 1/00, A61B 17/34, A61M 25/00

(54) **Transseptal needle apparatus**
Transseptale Nadelvorrichtung
Appareil à aiguille transseptale

(30) Priority: 08.05.2012 US 201261644180 P
(43) Date of publication of application: 13.11.2013
(73) Proprietor: Greatbatch Ltd., Clarence, NY 14031 (US)
(72) Inventor: Kimmel, Scott, St Paul, MN 55104 (US); Katra, Rodolphe, Blaine, MN 55449 (US)
(74) Representative: Garratt, Peter Douglas

(56) References cited:
- EP-A1- 1 462 141
- EP-A2- 0 537 573
- WO-A1-00/56238
- WO-A1-02/32335
- WO-A1-2005/104973
- WO-A2-2006/138462
- DE-A1- 10 004 385
- US-A- 5 578 052
- US-A- 5 861 002
- US-A- 6 063 081
- US-A1- 2002 183 738
- US-A1- 2003 187 460
- US-A1- 2008 161 792
- US-B1- 6 298 256

## Description

### CROSS REFERENCE TO RELATED APPLICATIONS

This application claims the benefit of priority to U.S. Provisional Application Serial No. 61/644,180, filed on May 8, 2012, entitled "TRANSSEPTAL NEEDLE".

### BACKGROUND

The present invention relates to transseptal catheterization, and more specifically relates to a radiofrequency (RF) assist needle for transseptal catheterization.

The human heart contains four chambers: the right atrium, the right ventricle, the left atrium, and the left ventricle. The right atrium is in fluid contact with the superior vena cava (SVC) and the inferior vena cava (IVC). The right atrium is separated from the right ventricle by the tricuspid valve, while the left atrium is separated from the left ventricle by the mitral valve. The right atrium and left atrium are separated by the interatrial septum, while the right ventricle and left ventricle are separated by the interventricular septum.

There are a multitude of therapeutic and diagnostic procedures in which a catheter is passed within a guide sheath or over a guide wire to access the chambers of the heart. The right atrium can be accessed from the superior vena cava or inferior vena cava. The right ventricle can be accessed from the right atrium. The left ventricle can be accessed from the aorta. The left atrium can be accessed directly from the left ventricle (through the mitral valve); however, such an approach is a relatively difficult maneuver due in part to the tortuous path that must be navigated with the catheter. Such a maneuver is problematic for various reasons, including a bleeding risk and a clotting risk to the patient (because it is the arterial side, it has a relatively high pressure, which exacerbates such risks). In addition, this approach may cause arrhythmias. Therefore, another approach for accessing the left atrium was developed. In this approach, a small hole is placed in the interatrial septum, so that the left atrium can be accessed from the right atrium. This hole is typically created by a needle puncture and is referred to as transseptal catheterization.

In the standard transseptal catheterization procedure, three separate tools are involved: a sheath with a sheath hub, a dilator with a dilator hub, and a needle assembly including a cannula, a needle hub and a stylet. The stylet is usually a small, guidewire-like device that is threaded through the needle cannula and attaches to the needle hub proximally. The distal tip of the stylet extends beyond the distal tip of the needle. The distal section of the needle has a shoulder or tapered section that corresponds with an internal taper at the distal tip of the dilator. When the needle is fully inserted into the dilator, the needle shoulder functions as a hard stop that limits the distance that the tip of the needle can exit from the dilator.

The typical transseptal procedure entails numerous steps. First, right femoral vein access is gained via the Seldinger technique. Second, a guidewire is passed through an introducer sheath, which was placed in the first step, into the femoral vein and threaded up the IVC to the SVC. Third, a sheath and dilator assembly is maneuvered to the SVC by being passed over the guidewire. Fourth, the guidewire is removed. Fifth, the needle assembly, usually including the stylet, is advanced through the inner lumen of the dilator until the distal tip of the needle (or stylet) is just proximal of the distal tip of the dilator. Sixth, the stylet, if present, is removed, and the needle is advanced until the tip of the needle is just proximal of the distal tip of the dilator. Seventh, the dilator/sheath/needle assembly is pulled caudally until the distal tip of the dilator is just resting on the fossa ovalis, which is a relatively thin area in the interatrial septum. Eighth, the needle is advanced forward through the dilator to puncture the septal wall (fossa ovalis). Ninth, and finally, the sheath and dilator assembly is fed through the septal wall over the needle, thereby gaining access to the left atrium.

One risk associated with transseptal needle use occurs if the patient presents with either a fibrotic or aneurismal septum. In the case of the fibrotic septum, the septum is very thick. In the case of the aneurismal septum, the septum is very thin. Both cases are difficult to puncture with the standard transseptal needle. Physicians typically do not know how successful the transseptal procedure will be until they perform it, especially in cases of aneurismal or fibrotic septums. If a standard mechanical transseptal puncture fails, physicians may need to replace the mechanical needle with an RF needle, which can be relatively expensive, in order to complete the procedure.

WO 02/32335 describes an apparatus for detecting and treating tumors using localized impedance measurement. The apparatus comprises an elongated delivery device that includes a lumen and is maneuverable in tissue. An impedance sensor array is deployable from the elongated delivery device and configured to be coupled to at least one of an electromagnetic energy source or a switching device. The impedance array includes a plurality of resilient members, at least one resilient member of the plurality of resilient members being positionable in the elongated delivery device in a compacted state and deployable with curvature into tissue from the elongated delivery device in a deployed state. In the deployed state, the plurality of resilient members defines a sample volume. At least one resilient member includes an impedance sensor and at least a portion of the impedance array is configured to sample tissue impedance through a plurality of conductive pathways. An energy delivery device is coupled to one of the sensor array, the at least one resilient member or the elongated delivery device.

US 5,861,002 describes an endoscopic surgical instrument treatment device assembly for inserting an endoscope and hollow core electrode assembly through a single access conduit, including a curved electrode guiding sheath or bendable bellows mechanism to deflect the electrode at an angle to the primary axis of the instrument. The device assembly further includes an electrode/needle supply connector assembly through which treatment fluids, laser fiberoptics or microsurgical instruments can be inserted into and through the hollow core electrode. A connector assembly is included for connecting the electrode with an RF energy source for operation in monopolar mode, or for connection of the RF power supply active side to the electrode and RF supply passive side to an electrode guiding sheath additionally functioning as an electrical return path.

US 2003/187460 describes apparatus and surgical methods which establish temporary suction attachment to a target site on the surface of a bodily organ for enhancing accurate placement of a surgical instrument maintained in alignment with the suction attachment. A suction port on the distal end of a supporting cannula provides suction attachment to facilitate accurate positioning of a needle for injection penetration of tissue at the target site on the moving surface of a beating heart. Force applied via the suction attachment to the surface of the heart promotes perpendicular orientation of the surface of the myocardium for enhanced accuracy of placement of a surgical instrument thereon.

US 6,063,081 describes an electrocautery instrument with a hollow electrode having a source of conductive fluid coupled to a proximal end thereof. Conductive fluid is communicated through said electrode and expelled out of the distal end thereof during electrocautery, forming a "virtual electrode." The infused conductive liquid conducts the RF electrocautery energy away from the conductive electrode, thereby displacing the region of thermal generation and reducing the extent of burns and perforations caused by conventional electrocautery electrodes. In one embodiment, the electrode is partially disposed within and extends distally out of a retractable suction tube, such that smoke and fluid are aspirated from the electrocautery site. When the suction tube is fully advanced, the electrode is concealed therein, enabling suction without electrocautery to be performed.

US 5,578,052 describes a laparoscopic instrument formed by a removable shaft combination portion separable from a housing. The instrument has a plug for connecting to an electric cord to allow the instrument to be used in cauterization techniques.

DE 100 04 385 A1 describes a surgical electrode apparatus for coagulating and/or selecting organic tissues, which is provided with a suction and rinsing device. The device includes a handle section with a connecting and operating member designed in two parts. A connecting part is provided at the end of the handle section with a connection tube and a Luer lock adapter, and a separate RF connection element is designed as a connection body penetrating the handle section at an acute angle. The connection body has a guide bore for a contact pin which is movable and fixable therein.

WO 00/56238 describes an adapter unit for converting a mechanical cutting device to an electrosurgical cutting device. The unit includes a mounting block from which an electrically insulating sheath extends. The sheath fits over the shaft of the cutting implement, is secured in alignment, attached so that the cutting tip, and its window are exposed through the sheath. A conductor extends along the length of the sheath, and is exposed to form a distal electrode, at the tip in close proximity to the opening, constituting one electrode of a bipolar electrode arrangement at the exposed tool. The other electrode is provided by electrical connection to the implement itself, so that a high current density path is formed through tissue in the cutting region.

US 6,298,256 describes a device for the location and catheterization of the surrounding area of a nerve of a human or animal body, including a catheter and an electrically conducting puncture needle with proximal and distal ends, which puncture needle forms a continuous lumen with its proximal and distal end open and having an inside diameter which at least corresponds to the outside diameter of the catheter and which, in the region of its proximal end, is equipped with an electrical connecting part for the connection of the puncture needle to an electrical voltage source. At the proximal end, a connecting element with a through-hole communicating with the lumen is provided, onto which an injection tube for the introduction of a liquid through the through-hole into the lumen of the puncture needle is connected, and through the through-hole of which the catheter can be pushed forward into the lumen of the puncture needle all the way to the distal end thereof.

US 2002/183738 describes a method and apparatus for treatment of atrial fibrillation. One exemplary embodiment provides a method of treating cardiac arrhythmia. This method includes positioning a distal tissue-contacting portion of a body in surface contact with a tissue surface of cardiac tissue; detecting the surface contact between the tissue-contacting portion and the tissue surface; and thereafter, injecting a tissue-ablating agent into the cardiac tissue through the tissue-contacting portion of the body.

### OVERVIEW

This overview is intended to provide an overview of subject matter of the present patent application. It is not intended to provide an exclusive or exhaustive explanation of the invention. The detailed description is included to provide further information about the present patent application.

The present inventors have recognized, among other things, that the subject matter can be used to selectively assist in puncturing a tissue layer, for instance, puncturing of a septal wall during transseptal catheterization. The present inventors have further recognized, among other things, that the subject matter can be used to inhibit formation of an embolus or inhibit the release of an embolus into the bloodstream.

### BRIEF DESCRIPTION OF THE DRAWINGS

FIG. 1A is a perspective view of a proximal end of a transseptal needle assembly in accordance with at least one example of the invention.
FIG. 1B is a perspective view of a proximal end of a transseptal needle assembly in accordance with at least one example of the invention.
FIG. 1C is a perspective view of a distal end of a transseptal needle assembly in accordance with at least one example of the invention.
FIG. 2 is a perspective view of an electrocautery device in use with a transseptal needle assembly of the present invention in accordance with at least one example of the invention.
FIG. 3A is a perspective view of a portion of a handle of a transseptal needle assembly in accordance with at least one example of the invention.
FIG. 3B is a perspective view of an electrocautery device in use with the portion of the handle of FIG. 3A in accordance with at least one example of the invention.
FIG. 4 is a perspective view of an electrocautery device in use with a transseptal needle assembly of the present invention in accordance with at least one example of the invention.
FIG. 5 is a perspective view of an electrocautery device in use with a transseptal needle assembly of the present invention in accordance with at least one example of the invention.
FIG. 6 is a perspective view of a distal end of a transseptal needle assembly, the distal end including a plug in accordance with at least one example of the invention.
FIG. 7 is a perspective view of a proximal end of a transseptal needle assembly in accordance with at least one example of the invention.
FIG. 8A is a perspective view of a proximal end of a transseptal needle assembly showing an actuator for an actuatable plug in accordance with at least one example of the invention.
FIG. 8B is a cross sectional view of a distal end of a transseptal needle assembly showing an actuatable plug in a distal position in accordance with at least one example of the invention.
FIG. 9A is a perspective view of a proximal end of a transseptal needle assembly showing an actuator for an actuatable plug in accordance with at least one example of the invention.
FIG. 9B is a cross sectional view of a distal end of a transseptal needle assembly showing an actuatable plug in a proximal position in accordance with at least one example of the invention.

### DETAILED DESCRIPTION

The present patent application discloses apparatuses for selectively providing assistance in puncturing a tissue layer, such as a septal wall, with a needle assembly, such as a transseptal needle assembly, or to inhibit formation of an embolus or releasing of an embolus into the bloodstream during such puncturing of a tissue layer. For instance, the apparatuses are used, in some examples, to selectively use radiofrequency energy to assist in puncturing a septal wall. In some examples, the apparatuses include one or more anti-embolus features, which inhibit formation of emboli or release of emboli into the bloodstream. The apparatuses, in some examples, are used in various procedures, including, but not limited to, tissue puncture procedures, interatrial septum puncture procedures, or transseptal catheterization procedures.

In various examples, the present disclosure includes apparatuses which assist physicians or other users to puncture a tissue layer, such as a septum, when mechanical puncturing means alone are ineffective. For instance, a fibrotic or aneurismal septum can prove difficult to puncture with only a mechanical transseptal needle. However, in some examples, a transseptal needle assembly includes a handle including an electrocautery receiver configured to selectively receive an electrocautery device to allow the physician or other user to use an electrocautery device to assist in puncturing a tissue layer, such as a septum. In some examples, the electrocautery receiver allows the physician or other user to selectively apply radiofrequency (RF) energy to and conduct RF energy through a cannula with an RF energy source, such as, for instance, an electrocautery pen. This allows physicians to selectively burn or cut a hole in a tissue layer, for instance, should a fibrotic or aneurismal septum be encountered, without having to switch from a mechanical needle to an RF needle.

In some examples, the present patent application includes apparatuses with an anti-embolus feature, which inhibits formation or release of an embolus into the bloodstream. In some examples, such an embolus can form from tissue or blood charring from using RF energy to assist in puncturing of tissue with a needle. In other examples, an embolus can form using just a mechanical needle to puncture tissue (for instance, coring of the tissue). That is, in various examples, examples of the anti-embolus feature can be used in addition to or separate from the puncture assist examples (for instance, the RF assist examples).

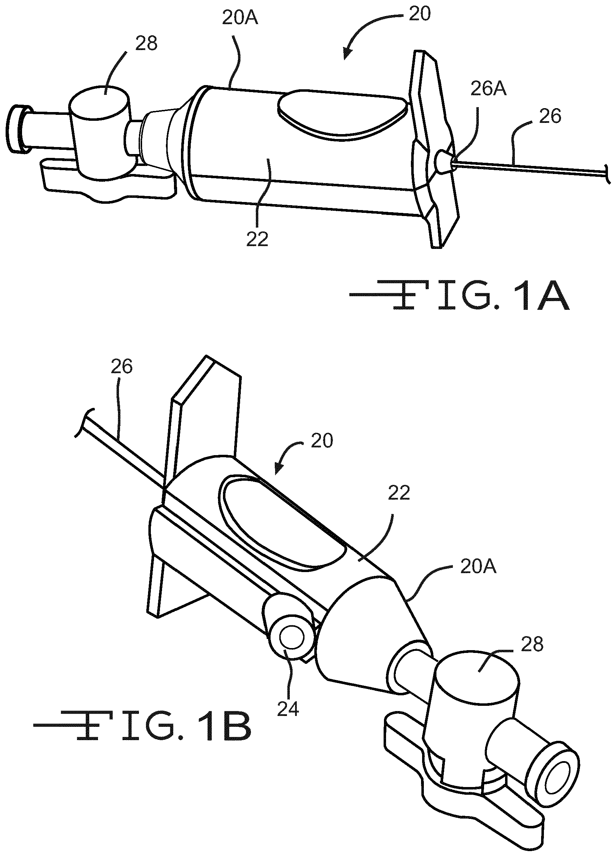

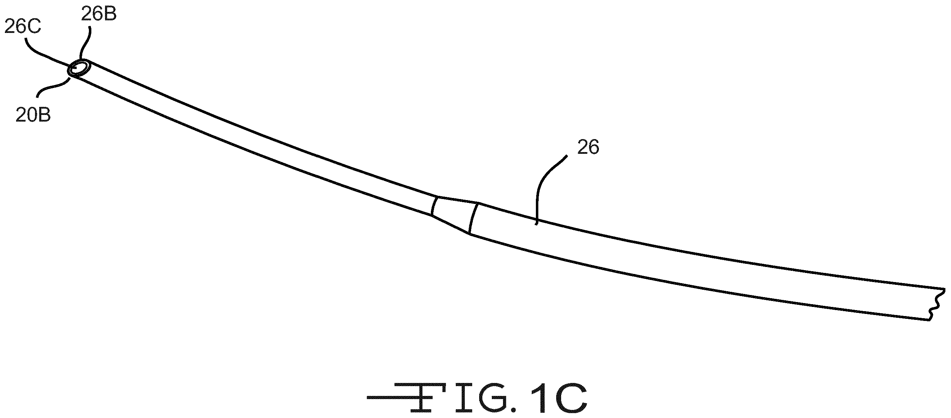

Referring to FIGS. 1A-1C, an apparatus 20, in various examples, includes a transseptal needle assembly 20. Although a transseptal needle assembly is primarily described herein, this is merely for the sake of simplicity of description and is not intended to be limiting. As such, it should be understood that the description presented herein can be applied to other apparatuses, such as other types of needle assemblies or cannula configurations.

In some examples, the transseptal needle assembly 20 includes a proximal end 20A and a distal end 20B. At the proximal end 20A, the transseptal needle assembly 20, in some examples, includes a handle 22. In some examples, the transseptal needle assembly 20 includes a needle cannula 26 extending from the handle 22. The needle cannula 26, in some examples, includes a proximal end 26A engaged with the handle 22 and a distal end 26B configured to puncture a tissue layer. In various examples, a portion of the proximal end 26A of the needle cannula is attached to the handle 22. In some examples, a portion of the proximal end 26A is disposed within the handle 22. In some examples, the proximal end 26A is non-detachably retained within the handle 22, for instance, being molded within the handle 22. In other examples, the needle cannula 26 can be detachably coupled to the handle 22 using a threaded coupling or other such mating connector coupling. In some examples, the needle cannula 26 includes a lumen 26C extending from the proximal end 26A to the distal end 26B.

Referring now to FIGS. 1A-2, in various examples, the transseptal needle assembly 20 includes an electrocautery receiver 24 for selectively accepting an electrocautery device 32. In some examples, the electrocautery receiver 24 is associated with the handle 22 and is electrically coupled to the needle cannula 26. In some examples, the electrocautery device 32 includes an energy source. In further examples, the electrocautery device 32 includes a radiofrequency (RF) energy source. In some examples, the electrocautery device 32 includes a Bovie pen. In various examples, the electrocautery receiver 24 is configured to selectively receive a portion of the electrocautery device 32 or energy (for instance, RF energy) from the electrocautery device 32 to allow the energy to be conducted from the electrocautery device 32 to the needle cannula 26. In this way, a physician or other user can selectively use the electrocautery device 32 to assist in puncturing a tissue layer (for instance, an interatrial septum) with the needle cannula 26 by conducting electrical energy (such as, for instance, RF energy) through the needle cannula 26 to the distal end 26B of the needle cannula 26. In some examples, the needle cannula 26 is formed from a conductive material. In further examples, the needle cannula 26 is formed from metal. In still further examples, the needle cannula 26 is formed from stainless steel. That is, if the physician or other user needs or wants assistance in puncturing a tissue layer, the physician or other user can place at least a portion of the electrocautery device 32 within or in contact with the electrocautery receiver 24 and activate the electrocautery receiver 24 to conduct electrical energy from the electrocautery device 32 to the distal end 26B of the needle cannula 26 to selectively electrocauterize tissue in contact with the distal end 26B of the needle cannula 26. In some examples, the electrical energy conducted from the electrocautery device 32 to the distal end 26B of the needle cannula 26 includes radiofrequency energy. By electrocauterizing the tissue in contact with the distal end 26B of the needle cannula 26, in some examples, the tissue is burned or cut to assist the distal end 26B of the needle cannula 26 to puncture the tissue (for instance, the interatrial septum). In some examples, such assistance in puncturing tissue can be desirable if the tissue is proving to be difficult to mechanically puncture using just the needle cannula 26. Such conditions can occur, for instance, if the tissue is fibrotic or aneurismal, in addition to other reasons.

In some examples, the handle 22 includes an insulating material, for instance, to guard against injury of the physician or other user or the patient during use of the electrocautery device 32 with the transseptal needle assembly 20. In some examples, the needle cannula 26 is insulated to guard against injury of the physician or other user or the patient during use of the electrocautery device 32 with the transseptal needle assembly 20. In further examples, a length of the needle cannula 26 is insulated, leaving the distal end 26B uninsulated to lessen the likelihood of charring tissue, fluids, or the like along the length of the needle cannula 26 but allowing the distal end 26B to impart energy (for instance, RF energy) to the tissue to selectively burn or cut the tissue in contact with the distal end 26B and assist in puncturing of the tissue with the needle cannula 26. In some examples, the needle cannula 26 includes insulating material covered, coated, or otherwise applied to an outside surface of the needle cannula 26.

In some examples, the transseptal needle assembly 20 includes a stopcock 28 or other coupling assembly attached to the handle 22 or otherwise fluidly coupled with the lumen 26C of the needle cannula 26. In various examples, the stopcock 28 or other coupling assembly can be used to fluidly couple to devices, such as, but not limited to, a syringe 30, a positive pressure source, a negative pressure source, or a combination thereof. In some examples, the physician or other user can couple the syringe 30 to the stopcock 28 or other coupling assembly, for instance, to pass a substance down the lumen 26C of the needle cannula 26 and into the patient. In some examples, the syringe 30 can be used to inject a contrast agent into the patient, for instance, to better visualize the tissue being punctured under fluoroscopy. In other examples, the positive pressure source or the negative pressure source can be coupled to the stopcock 28 or other couple assembly in order to pass a fluid or other material out of the needle cannula 26 or draw a fluid or other material into the needle cannula 26, respectively. In further examples, the stopcock 28 or other coupling assembly can be configured to selectively couple to more than one device at a time, thereby enabling, for instance, a combination of the syringe 30, the positive pressure source, and the negative pressure source to be coupled to the transseptal needle assembly 20 at the same time. It is contemplated in some examples that the stopcock 28 or other coupling assembly be configured to allow only one of the connected devices to actually fluidly couple to the needle cannula 26. In this way, some or all of the devices can be attached to the transseptal needle assembly 20 at once. Such a configuration allows the physician or other user to just start operating the desired device rather than having to disconnect one device and/or connect another device prior to using the desired device, while also inhibiting the various devices from competing against each other (for instance, the positive and negative pressure sources counteracting each other or the negative pressure source preventing the contrast agent from the syringe 30 making it into the patient). In various examples, the stopcock 28 or other coupling device can include a selector control to allow the physician or other user to select which device coupled to the stopcock 28 or other coupling assembly to fluidly couple to the needle cannula 26.

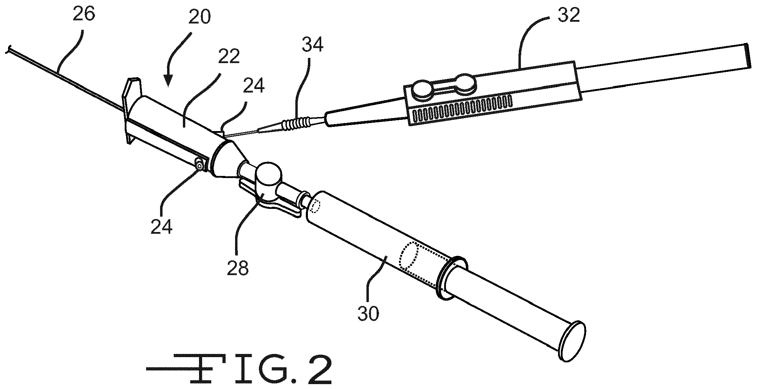

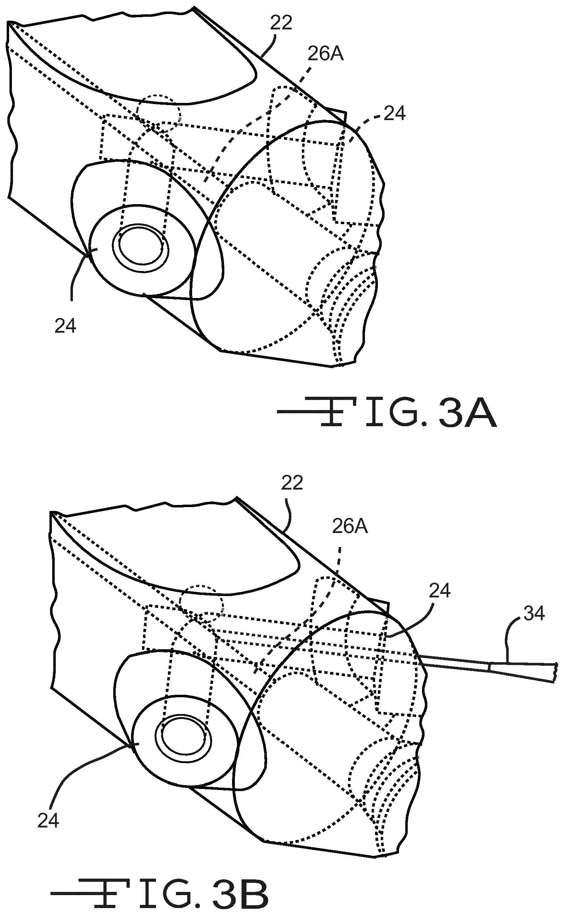

Referring now to FIGS. 1A-3B, in some examples, the electrocautery receiver 24 includes a port 24 in the handle 22. In further examples, the port 24 is sized to receive a portion (for instance, a tip 34) of the electrocautery device 32 to allow the electrocautery device 32 to directly contact a portion of the proximal end 26A of the needle cannula 26 (FIG. 3B). In some examples, the transseptal needle assembly 20 includes one port 24 (FIGS. 1A and 1B). In some examples, the transseptal needle assembly 20 includes at least two ports 24 (FIGS. 2-3B). In further examples, the transseptal needle assembly 20 includes more than two ports 24. In further examples, each of the ports 24 is located at an angular location in the handle 22 that is different from the angular location of each of the other one or more ports 24. In still further examples, at least two of the at least two ports 24 are diametrically opposed to one another. By having the ports 24 located a various angular locations around the handle 22, the physician or other user has multiple locations in which to insert the electrocautery device 32 to decrease the likelihood that the port 24 is at an inconvenient location or angle if or when the physician or other user inserts the electrocautery device 32 during the procedure.

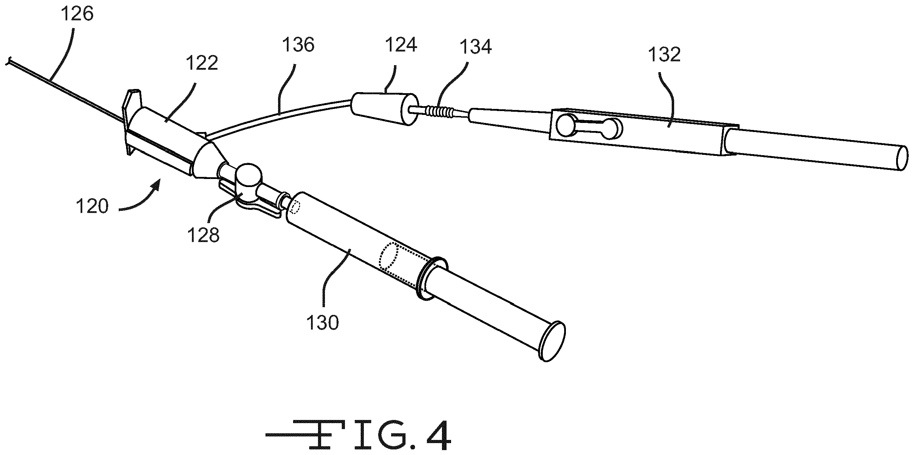

Referring to FIG. 4, in some examples, a transseptal needle assembly 120 is substantially similar to the transseptal needle assembly 20 described herein. In some examples, a handle 122 of the transseptal needle assembly 120 includes an electrocautery receiver 124 that includes a receptacle 124 spaced from the handle 122. In some examples, the receptacle 124 extends out of the handle 122 and is integrally attached to the handle 122. That is, the receptacle 124 includes a protrusion that extends outwardly a distance from the handle 122, such that the receptacle 124 is rigidly attached to the handle 122. In other examples, the receptacle 124 is non-rigidly attached to the handle 122. For instance, in some examples, the receptacle 124 is attached to the handle 122 using a wire 136. That is, in various examples, the receptacle 124 can be movable with respect to the handle 122. In some examples, the receptacle 124, whether rigidly or non-rigidly attached to the handle 122 can be electrically coupled to a needle cannula 126 with the wire 136 (whether the wire 136 is within the rigid protrusion or flexibly coupling the receptacle 124 to the handle 122). In either situation, the receptacle 124 can include a contact surface within the receptacle 124 electrically coupled to the wire 136 to allow energy to selectively conduct from an electrocautery device 132 through the wire 136 and to the needle cannula 126 if or when the physician or other user wants assistance from the electrocautery device 132 in puncturing tissue. In some examples, the energy selectively conducted from the electrocautery device 132 includes electrical energy. In further examples, the energy selectively conducted from the electrocautery device 132 includes RF energy. The transseptal needle assembly 120, in some examples, further includes a stopcock 128 or other coupling assembly, substantially similar to the stopcock 28 or other coupling assembly described herein, attached to the handle 122 or otherwise fluidly coupled with the lumen of the needle cannula 126 for coupling to devices, such as, but not limited to, a syringe 130, a positive pressure source, a negative pressure source, or a combination thereof.

In some examples, the handle 122 includes an insulating material, for instance, to guard against injury of the physician or other user or the patient during use of the electrocautery device 132 with the transseptal needle assembly 120. In some examples, the needle cannula 126 is insulated to guard against injury of the physician or other user or the patient during use of the electrocautery device 132 with the transseptal needle assembly 120. In further examples, a length of the needle cannula 126 is insulated, leaving the distal end uninsulated to lessen the likelihood of charring tissue, fluids, or the like along the length of the needle cannula 126 but allowing the distal end to impart energy (for instance, RF energy) to the tissue to selectively burn or cut the tissue in contact with the distal end and assist in puncturing of the tissue with the needle cannula 126. In some examples, the needle cannula 126 includes insulating material covered, coated, or otherwise applied to an outside surface of the needle cannula 126.

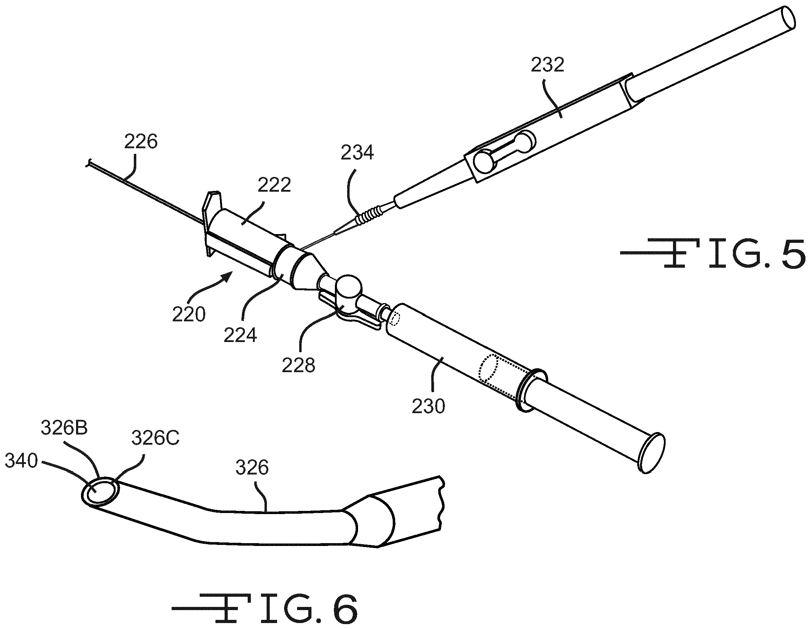

Referring to FIG. 5, in some examples, a transseptal needle assembly 220 is substantially similar to the transseptal needle assemblies 20, 120 described herein. In some examples, a handle 222 of the transseptal needle assembly 220 includes an electrocautery receiver that includes a conductive surface 224 electrically coupled to a needle cannula 226. In some examples, the conductive surface 224 is configured to be contacted by an electrocautery device 232. That is, the conductive surface 224, in some examples, is located on the handle 222 and is accessible to be selectively contacted by a portion 234 of the electrocautery device 232. In some examples, the conductive surface 224 includes an annular surface disposed on the handle 222. In further examples, the handle 222 includes more than one conductive surface disposed at various locations around the handle 222 to allow the physician or other user to contact one of the conductive surfaces at various angles. In some examples, the conductive surface 224 includes a ring disposed around the handle 222. Such a configuration allows access to the conductive surface 224 from multiple angles to allow for the physician or other user to selectively contact the conductive surface 224 to lessen the likelihood of the orientation of the handle 222 causing obstruction of the conductive surface 224 during a puncturing procedure. In various examples, the conductive surface 224 can be recessed with respect to a surface of the handle 222 to safeguard against inadvertent contacting of the conductive surface 224 with the electrocautery device 232. In other examples, the conductive surface 224 can include a protective covering which must be slid back, opened, removed, or the like prior to use with the electrocautery device 232 to safeguard against inadvertent contacting of the conductive surface 224 with the electrocautery device 232. Contacting of the electrocautery device 232 with the conductive surface 224 electrically couples the electrocautery device 232 to the wire needle cannula 226 to allow energy to selectively conduct from the electrocautery device 132 to the needle cannula 226 if or when the physician or other user wants assistance from the electrocautery device 232 in puncturing tissue. In some examples, the energy selectively conducted from the electrocautery device 232 includes electrical energy. In further examples, the energy selectively conducted from the electrocautery device 232 includes RF energy. The transseptal needle assembly 220, in some examples, further includes a stopcock 228 or other coupling assembly, substantially similar to the stopcock 28, 128 or other coupling assembly described herein, attached to the handle 222 or otherwise fluidly coupled with the lumen of the needle cannula 226 for coupling to devices, such as, but not limited to, a syringe 230, a positive pressure source, a negative pressure source, or a combination thereof.

In some examples, the handle 222 includes an insulating material, for instance, to guard against injury of the physician or other user or the patient during use of the electrocautery device 232 with the transseptal needle assembly 220. In some examples, the needle cannula 226 is insulated to guard against injury of the physician or other user or the patient during use of the electrocautery device 232 with the transseptal needle assembly 220. In further examples, a length of the needle cannula 226 is insulated, leaving the distal end uninsulated to lessen the likelihood of charring tissue, fluids, or the like along the length of the needle cannula 226 but allowing the distal end to impart energy (for instance, RF energy) to the tissue to selectively burn or cut the tissue in contact with the distal end and assist in puncturing of the tissue with the needle cannula 226. In some examples, the needle cannula 226 includes insulating material covered, coated, or otherwise applied to an outside surface of the needle cannula 226.

In various examples, conducting energy (for instance, RF energy) along the needle cannula 26, 126, 226 allows for selective burning of tissue. However, there is also the chance of charring of blood or other tissue located within the hollow needle cannula 26, 126, 226, which could potentially result in an embolus being released into the patient. While this potential exists with respect to using the transseptal needle assembly 20, 120, 220 with the electrocautery device 32, 132, 232, the potential of embolus creation also exists when using the transseptal needle assembly 20, 120, 220 without the electrocautery device 32, 132, 232. For instance, the needle cannula 26, 126, 226 can core tissue that it punctures, essentially creating a plug of tissue within the needle cannula 26, 126, 226 that can potentially be released into the patient as an embolus. For at least these reasons, the present description further contemplates anti-embolus features, as described herein, associated with the transseptal needle assembly 20, 120, 220. These anti-embolus features, in some examples, can be used in conjunction with the described configurations for selectively using energy (for instance, RF energy) to assist in puncturing of tissue. However, these anti-embolus features, in other examples, can be used in independently from the described configurations for selectively using energy (for instance, RF energy) to assist in puncturing of tissue. That is, the anti-embolus features described herein are not required to be used with transseptal needle assemblies 20, 120, 220 including the electrocautery receivers 24, 124, 224, and can be used either with such transseptal needle assemblies 20, 120, 220 including the electrocautery receivers 24, 124, 224 (or otherwise the ability to selectively assist puncturing using energy, electrical energy, or RF energy) or with transseptal needle assemblies not including electrocautery receivers (or otherwise the ability to selectively assist puncturing using energy, electrical energy, or RF energy).

Referring to FIG. 6, in some examples, a distal end 326B of a needle cannula 326 includes an anti-embolus feature 340. In some examples, the anti-embolus feature 340 includes a plug 340 disposed within a lumen 326C of the needle cannula 326. In some examples, the plug 340 is disposed at the distal end 326B of the needle cannula 326 to essentially cap off the lumen 326C at the distal end 326B of the needle cannula 326 to inhibit materials (such as tissue, blood, or the like) from entering the lumen 326C of the needle cannula 326. In some examples, the plug 340 is disposed within the lumen of the needle cannula 326 and is movable within the lumen 326 between a first position disposed proximate the distal end 326B of the needle cannula 326 and a second position spaced proximally from the first position. In this way, the plug 340 can inhibit the coring of tissue or the burning or charring of tissue, blood, or another substance within the lumen 326C (if selectively using energy, electrical energy, or RF energy with the needle cannula 326, for instance, as described with respect to transseptal needle assemblies 20, 120, 220 herein) by inhibiting access of such tissue, blood, or other substances within the lumen 326C. Because it can be desirable to selectively open the lumen 326C of the needle cannula 326 (for instance, in order to inject contrast material through the lumen 326C and into the patient), in some examples, the plug 340 is removable from or otherwise actuatable within the lumen 326C. In some examples, the plug 340 includes a removable tube removably disposed within the lumen 326. In some examples, the removable tube includes a polymeric material. In some examples, the plug 340 is a tip of an elongate column of material, a proximal end of the column being accessible to the physician or other user from a proximal end of the transseptal needle assembly to allow the physician or other user to remove the column from within the lumen 326C (and, in turn, the plug 340 from the distal end 326B) from the proximal end of the transseptal needle assembly. In other examples, the plug 340 is smaller (for instance, it does not extend all the way to the proximal end of the transseptal needle assembly as did the column), such that it is disposed at the distal end 326B and is selectively movable proximally within the lumen 326C in order to open the distal end 326B of the needle cannula 326 (for instance, to allow injecting of contrast material into the patient). The needle cannula 326, in some examples, can be configured to allow movement of the plug 340 from a distal, closed position to a proximal, open position. In some examples, application of negative pressure within the needle cannula 326 can draw the plug 340 back to the proximal, open position. In some examples, application of positive pressure within the needle cannula 326 can push the plug 340 to (and maintain the plug 340 in) the distal, closed position. In some examples, an interior wall of the lumen 326C includes stops or other features to define the open and closed positions of the plug 340 and to inhibit the plug 340 from exiting from the distal end 326B of the needle cannula 326 and entering the patient or from being drawn proximally through the needle cannula 326 too far. In various examples, the plug 340 can include various shapes, including, but not limited to, substantially cylindrical, substantially spherical, or another shape capable of traversing within the lumen 326C between the closed and open positions.

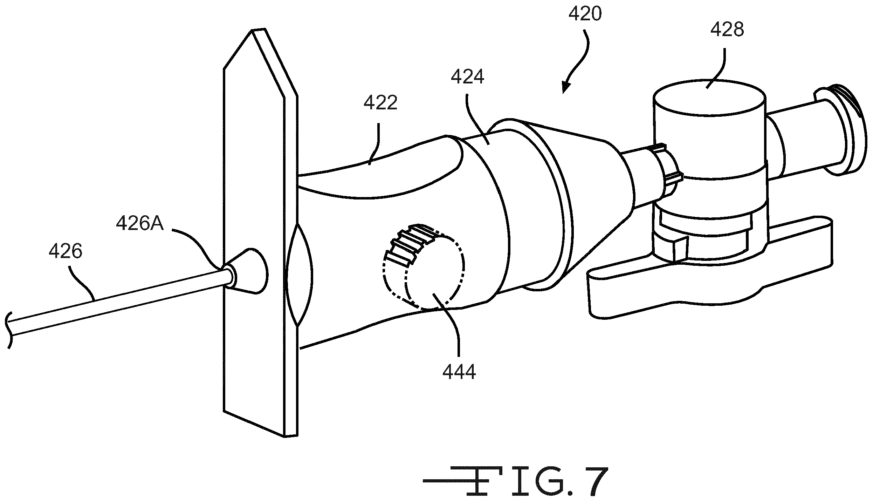

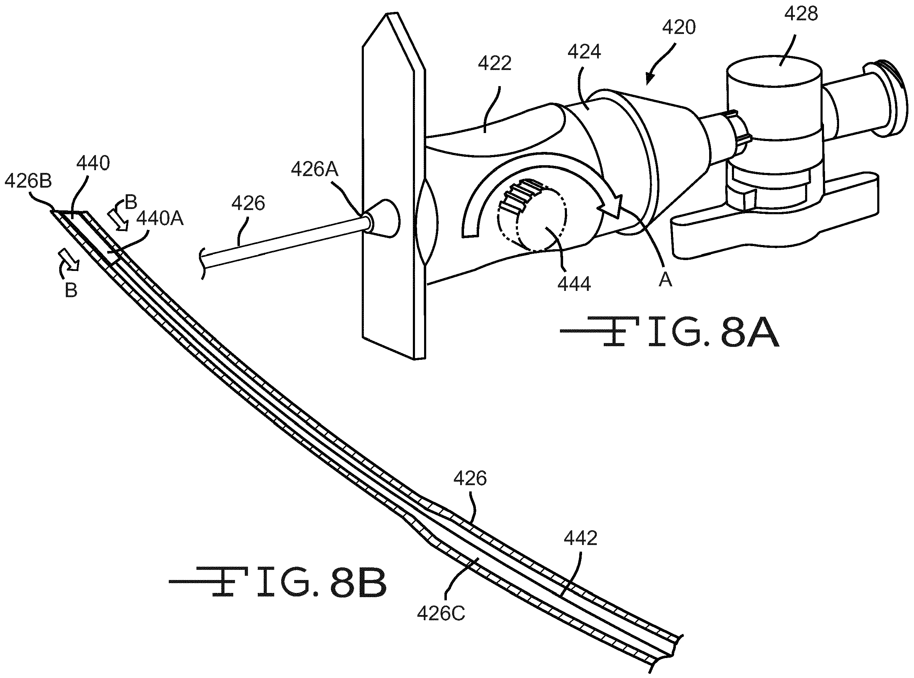

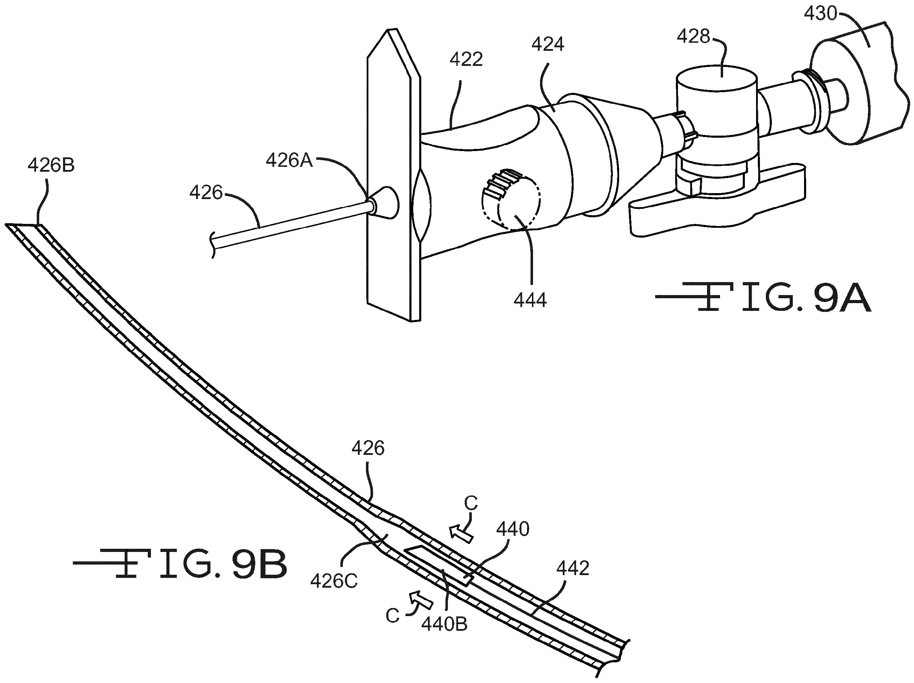

Referring to FIGS. 7-9B, a transseptal needle assembly 420 can be substantially similar to the transseptal needle assemblies 20, 120, 220 described herein. Although shown with an electrocautery receiver 424 similar to the electrocautery receiver 224 described herein, it is contemplated that the electrocautery receiver 424 will be similar to other electrocautery receivers described herein (such as, for instance, the electrocautery receivers 24, 124) or that the transseptal needle assembly 420 include no electrocautery receiver. In some examples, the transseptal needle assembly 420 includes an anti-embolus feature including an actuatable plug 440 disposed within a lumen 426C of a needle cannula 426. In some examples, the actuatable plug 440 is movable within the lumen 426C between a first position 440A (FIG. 8B) disposed proximate a distal end 426B of the needle cannula 426 and a second position 440B (FIG. 9B) spaced proximally from the first position 440A. As described herein with respect to the plug 340, in some examples, such actuation of the actuatable plug 440 to the first position 440A can be selectively implemented to substantially close off the distal end 426B of the needle cannula 426 to inhibit coring of tissue, burning or charring of tissue, blood, or other substances within the lumen 426C (if selectively using energy, electrical energy, or RF energy with the needle cannula 426, for instance, as described with respect to transseptal needle assemblies 20, 120, 220 herein), or the like by inhibiting access of such tissue, blood, or other substances within the lumen 426C. In some examples, such actuation of the actuatable plug 440 to the second position 440B can be selectively implemented to substantially open up the distal end 426B of the needle cannula 426 (for instance, in order to inject contrast material through the lumen 426C and into the patient). As shown in FIG. 9B, in some examples, the actuatable plug 440 is disposed in a widened portion of the lumen 426C, which allows space for contrast material or other material to pass between a wall of the needle cannula 426 and the actuatable plug 440 in order to expel the material from the distal end 426B of the needle cannula 426. In various examples, actuation of the actuatable plug 440 between the first and second positions 440A, 440B can be accomplished in different manners.

In some examples, a handle 422 (or another aspect of the transseptal needle assembly 420) can include a plug control 444 operatively connected to the actuatable plug 440, actuation of the plug control 444 being configured to at least move the plug 440 in a first direction within the lumen 426C. In some examples, the plug control 444 includes a knob 444, such that rotation of the knob 444 in a first direction (for instance, rotation in the direction of arrow A of FIG. 8A) causes movement of the actuatable plug 440 in the direction of arrows B (FIG. 8B) to move the actuatable plug 440 from the first position 440A proximally toward the second position 440B. In some examples, the anti-embolus feature includes a tether 442 coupling the plug control 444 to the actuatable plug 440. That is, actuation of the plug control 444 pulls on the tether 442, which, in turn, pulls on the actuatable plug 440 to move the actuatable plug proximally. In some examples, the tether 442 is disposed within the lumen 426C of the needle cannula 426. In other examples, the tether can be disposed outside of the needle cannula 426. Although shown as a knob 444, it is contemplated that the actuator 444 include any suitable type of actuator, including, but not limited to a slider, a button, a toggle, or the like, provided it is capable of allowing the physician or other user to selectively control movement of the actuatable plug 440 in at least one direction. In some examples, movement of the actuatable plug 440 proximally toward the second position 440B can be accomplished using a negative pressure source to create negative pressure within the lumen 426C to draw the actuatable plug 440 proximally within the lumen 426C. Such use of negative pressure, in various examples, can be employed in addition to or instead of use of the plug control 444. In some examples, only the plug control 444 is used without also employing negative pressure.

In some examples, actuation of the plug control 444 (for instance, in an opposite direction to the arrow A of FIG. 8A) can allow the actuatable plug 440 to move distally toward the first position 440A. In some examples, movement of the actuatable plug 440 distally toward the first position 440A can be accomplished using a positive pressure source to create positive pressure within the lumen 426C to push the actuatable plug 440 distally within the lumen 426C. Such use of positive pressure, in various examples, can be employed in addition to or instead of use of the plug control 444. In some examples, only the plug control 444 is used without also employing positive pressure.

In some examples, an anti-embolus feature of the transseptal needle assembly 20, 120, 220, 420 includes a continuous pressure source fluidly coupled to the needle cannula 26, 126, 226, 326, 426 to inhibit formation of an embolus within or exiting of an embolus from within the lumen 26C, 126C, 226C, 326C, 426C of the needle cannula 26, 126, 226, 326, 426. In such examples, the substantially continuous movement of material into or out of the needle cannula 26, 126, 226, 326, 426 flushes the needle cannula 26, 126, 226, 326, 426 and inhibits formation of an embolus within or exiting of an embolus from within the lumen 26C, 126C, 226C, 326C, 426C.

In some examples, the anti-embolus feature includes a continuous positive pressure source fluidly coupled to the needle cannula 26, 126, 226, 326, 426 (for instance, using the stopcock 28, 128, 228, 428 or other coupling assembly) to inhibit formation of an embolus within the lumen 26C, 126C, 226C, 326C, 426C of the needle cannula 26, 126, 226, 326, 426. In some examples, a continuous flow of material (for instance, saline) can be selectively expelled from the needle cannula 26, 126, 226, 326, 426 using the positive pressure source. By flushing the lumen 26C, 126C, 226C, 326C, 426C of the needle cannula 26, 126, 226, 326, 426, the formation of an embolus (by coring of tissue, burning or charring of tissue, blood, or other substances within the lumen 26C, 126C, 226C, 326C, 426C (if selectively using energy, electrical energy, or RF energy with the needle cannula 426, for instance, as described with respect to transseptal needle assemblies 20, 120, 220 herein), or the like) is lessened by inhibiting materials (such as tissue, blood, or other materials) to enter the lumen 26C, 126C, 226C, 326C, 426C.

In some examples, the anti-embolus feature includes a continuous negative pressure source fluidly coupled to the needle cannula 26, 126, 226, 326, 426 (for instance, using the stopcock 28, 128, 228, 428 or other coupling assembly) to inhibit exiting of an embolus from within the lumen 26C, 126C, 226C, 326C, 426C of the needle cannula 26, 126, 226, 326, 426. In some examples, a continuous flow of material can be selectively drawn into the needle cannula 26, 126, 226, 326, 426 using the negative pressure source. By drawing material into the lumen 26C, 126C, 226C, 326C, 426C of the needle cannula 26, 126, 226, 326, 426, the formation of an embolus (by coring of tissue, burning or charring of tissue, blood, or other substances within the lumen 26C, 126C, 226C, 326C, 426C (if selectively using energy, electrical energy, or RF energy with the needle cannula 426, for instance, as described with respect to transseptal needle assemblies 20, 120, 220 herein), or the like) is lessened by inhibiting materials (such as tissue, blood, or other materials) to flow distally out of the lumen 26C, 126C, 226C, 326C, 426C and enter the patient.

The present inventors have recognized various advantages of the subject matter described herein. For instance, in some examples, the needle assemblies described herein can be used to puncture a tissue layer (for instance, a septal wall) and, if the tissue layer proves to be difficult to puncture through purely mechanical means, an electrocautery device can be used with the needle assembly already inserted within the patient to impart RF energy to the distal end of the needle cannula to assist with the puncturing of the tissue layer, wherein the RF energy assists in burning or cutting through the tissue layer. This can be done without removing the needle assembly or swapping the needle assembly for a specialized RF needle assembly, thereby saving time and cost of the procedure. In various examples, the needle assemblies described herein allow for selective RF assistance in puncturing a tissue layer with a decreased risk of harming the patient and the physician or other user performing the procedure. Additionally, in various examples, the needle assemblies described herein include anti-embolus features that inhibit emboli from forming or being released into the bloodstream, thereby decreasing the likelihood that an embolus will present a potentially dangerous situation for the patient. While various advantages of the example needle assemblies are listed herein, this list is not considered to be complete, as further advantages may become apparent from the description and figures presented herein.

Although the subject matter of the present patent application has been described with reference to various examples, workers skilled in the art will recognize that changes can be made in form and detail without departing from the scope of the subject matter recited in the below claims.

The above Detailed Description includes references to the accompanying drawings, which form a part of the Detailed Description. The drawings show, by way of illustration, specific examples in which the present apparatuses can be practiced. These embodiments are also referred to herein as "examples."

The above Detailed Description is intended to be illustrative, and not restrictive. For example, the above-described examples (or one or more elements thereof) can be used in combination with each other. Other embodiments can be used, such as by one of ordinary skill in the art upon reviewing the above description. Also, various features or elements can be grouped together to streamline the disclosure. This should not be interpreted as intending that an unclaimed disclosed feature is essential to any claim. Rather, inventive subject matter can lie in less than all features of a particular disclosed embodiment. Thus, the following claims are hereby incorporated into the Detailed Description, with each claim standing on its own as a separate embodiment. The scope of the invention should be determined with reference to the appended claims, along with the full scope of equivalents to which such claims are entitled.

In this document, the terms "a" or "an" are used to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "about" and "approximately" or similar are used to refer to an amount that is nearly, almost, or in the vicinity of being equal to a stated amount.

In the appended claims, the terms "including" and "in which" are used as the plain-English equivalents of the respective terms "comprising" and "wherein." Also, in the following claims, the terms "including" and "comprising" are open-ended, that is, an apparatus that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The Abstract is provided to allow the reader to quickly ascertain the nature of the technical disclosure. It is submitted with the understanding that it will not be used to interpret or limit the scope or meaning of the claims.

## Claims

1. An apparatus (20, 120, 220, 420) for selectively receiving an electrocautery device, the apparatus comprising:
a needle cannula (26, 126, 226, 326, 426) including a proximal end (26A) and a distal end (26B, 326B, 426B), the needle cannula (26, 126, 226, 326, 426) including a lumen (26C, 126C, 226C, 326C, 426C) extending from the proximal end (26A) to the distal end (26B, 326B, 426B);
a handle (22, 122, 222, 422) disposed at the proximal end (26A) of the needle cannula (26, 126, 226, 326, 426);
**characterised by** an electrocautery receiver (24, 124, 224, 424) associated with the handle (22, 122, 222, 422) and electrically coupled to the needle cannula (26, 126, 226, 326, 426), said electrocautery receiver (24, 124, 224, 424) comprising:
a port (24) in the handle (22), the port (24) configured to receive a portion (34) of the electrocautery device (32) to allow the electrocautery device (32) to directly contact a portion of the proximal end (26A) of the needle cannula (26); or
an annular conductive surface (224) electrically coupled to the needle cannula (226), the conductive surface (224) configured to be contacted by the electrocautery device (232); or
a receptacle (124) spaced from the handle (122) and configured to receive a portion (34) of the electrocautery device (32) the receptacle being electrically coupled to the needle cannula (126) with a wire (136) and being movable with respect to the handle;
such that electrical energy can be conducted from the electrocautery device (32, 132, 232) to the distal end (26B, 326B, 426B) of the needle cannula (26, 126, 226, 326, 426) to selectively electrocauterize tissue in contact with the distal end (26B, 326B, 426B) of the needle cannula (26, 126, 226, 326, 426).

2. The apparatus of claim 1, wherein the electrocautery receiver (24) includes a port (24) in the handle (22), the port (24) configured to receive a portion (34) of the electrocautery device (32) to allow the electrocautery device (32) to directly contact a portion of the proximal end (26A) of the needle cannula (26).

3. The apparatus of claim 2, wherein the electrocautery receiver includes at least two ports (24) in the handle (22).

4. The apparatus of claim 3, wherein each of the ports (24) is located at an angular location in the handle (22) that is different from the angular location of each of the other one or more ports (24).

5. The apparatus of claim 3 or claim 4, wherein at least two of the at least two ports (24) are diametrically opposed to one another.

6. The apparatus of claim 1, wherein the conductive surface includes a ring disposed around the handle (222).

7. The apparatus of any of claims 1 to 6, wherein the needle cannula (326, 426) is conductive to radio frequency electrical energy, and the apparatus includes an anti-embolus feature (340, 440) configured to inhibit formation during electrocautery of an embolus within or exiting of an embolus from within the lumen (326C, 426C) of the needle cannula (326, 426).

8. The apparatus of claim 7, wherein the anti-embolus feature (340, 440) includes a plug (340, 440) disposed within the lumen (326C, 426C) of the needle cannula (326, 426), the plug (340, 440) being movable within the lumen (326C, 426C) between a first position (440A) disposed proximate the distal end (326B, 426B) of the needle cannula (326, 426) and a second position (440B) spaced proximally from the first position (440A), the plug (340, 440) in the first position (440A) substantially occluding an opening at the distal end (326B, 426B) of the needle cannula (326, 426) to inhibit formation during electrocautery of an embolus within the lumen (326C, 426C) of the needle cannula (326, 426).

9. The apparatus of claim 7 or claim 8, wherein the anti-embolus feature (340, 440) includes a continuous pressure source fluidly coupled to the needle cannula (326, 426) to inhibit formation during electrocautery of an embolus within or exiting of an embolus from within the lumen (326C, 426C) of the needle cannula (326, 426).

10. The apparatus of claim 9, wherein the anti-embolus feature (340, 440) includes a continuous positive pressure source fluidly coupled to the needle cannula (326, 426) to inhibit formation during electrocautery of an embolus within the lumen (326C, 426C) of the needle cannula (326, 426).

11. The apparatus of claim 9 or claim 10, wherein the anti-embolus feature (340, 440) includes a continuous negative pressure source fluidly coupled to the needle cannula (326, 426) to inhibit exiting of an embolus from within the lumen (326C, 426C) of the needle cannula (326, 426).

## Patentansprüche

1. Vorrichtung (20, 120, 220, 420) zum selektiven Aufnehmen einer Elektrokauterisationsvorrichtung, wobei die Vorrichtung Folgendes umfasst:
eine Nadelkanüle (26, 126, 226, 326, 426) mit einem proximalen Ende (26A) und einem distalen Ende (26B, 326B, 426B), wobei die Nadelkanüle (26, 126, 226, 326, 426) ein Lumen (26C, 126C, 226C, 326C, 426C) umfasst, das sich von dem proximalen Ende (26A) zu dem distalen Ende (26B, 326B, 426B) erstreckt;
einen Griff (22, 122, 222, 422), der am proximalen Ende (26A) der Nadelkanüle (26, 126, 226, 326, 426) angeordnet ist;
**gekennzeichnet durch** einen Elektrokauterisationsempfänger (24, 124, 224, 424), der dem Handgriff (22, 122, 222, 422) zugeordnet und elektrisch mit der Nadelkanüle (26, 126, 226, 326, 426) verbunden ist, wobei der genannte Elektrokauterisationsempfänger (24, 124, 224, 424) Folgendes umfasst:
eine Öffnung (24) in dem Griff (22), wobei die Öffnung (24) dafür konfiguriert ist, einen Teil (34) der Elektrokauterisationsvorrichtung (32) aufzunehmen, um zu ermöglichen, dass die Elektrokauterisationsvorrichtung (32) direkt mit einer Öffnung des proximalen Endes (26A) der Nadelkanüle (26) in Kontakt kommt; oder
eine ringförmige leitfähige Oberfläche (224), die elektrisch mit der Nadelkanüle (226) verbunden ist, wobei die leitfähige Oberfläche (224) dafür konfiguriert ist, von der Elektrokauterisationsvorrichtung (232) kontaktiert zu werden; oder
ein Behältnis (124), das von dem Griff (122) beabstandet ist und dafür konfiguriert ist, einen Teil (34) der Elektrokauterisationsvorrichtung (32) aufzunehmen; wobei das Behältnis mit der Nadelkanüle (126) mit einem Draht (136) elektrisch verbunden ist und in Bezug auf den Griff beweglich ist;
so dass elektrische Energie von der Elektrokauterisationsvorrichtung (32, 132, 232) zum distalen Ende (26B, 326B, 426B) der Nadelkanüle (26, 126, 226, 326, 426) geleitet werden kann, um Gewebe in Kontakt mit dem distalen Ende (26B, 326B, 426B) der Nadelkanüle (26, 126, 226, 326, 426) selektiv zu elektrokauterisieren.

2. Vorrichtung nach Anspruch 1, wobei der Elektrokauterisationsempfänger (24) eine Öffnung (24) in dem Griff (22) umfasst, wobei die Öffnung (24) dafür konfiguriert ist, einen Teil (34) der Elektrokauterisationsvorrichtung (32) aufzunehmen; um zu ermöglichen, dass die Elektrokauterisationsvorrichtung (32) direkt mit einem Abschnitt des proximalen Endes (26A) der Nadelkanüle (26) in Kontakt kommt.

3. Vorrichtung nach Anspruch 2, wobei der Elektrokauterisationsempfänger mindestens zwei Öffnungen (24) in dem Griff (22) aufweist.

4. Vorrichtung nach Anspruch 3, wobei jede der Öffnungen (24) an einer Winkelstelle in dem Griff (22) angeordnet ist, die sich von der Winkelposition jeder der anderen einen oder mehreren Öffnungen (24) unterscheidet.

5. Vorrichtung nach Anspruch 3 oder Anspruch 4, wobei mindestens zwei der mindestens zwei Öffnungen (24) einander diametral gegenüberliegen.

6. Vorrichtung nach Anspruch 1, wobei die leitfähige Oberfläche einen Ring umfasst, der um den Griff (222) herum angeordnet ist.

7. Vorrichtung nach einem der Ansprüche 1 bis 6, wobei die Nadelkanüle (326, 426) für elektrische Hochfrequenzenergie leitfähig ist und die Vorrichtung ein Antiembolusmerkmal (340, 440) umfasst, das dafür konfiguriert ist, die Bildung eines Embolus innerhalb des Lumens (326C, 426C) der Nadelkanüle (326, 426) oder das Austreten eines Embolus aus dem Lumen (326C, 426C) der Nadelkanüle (326, 426) während der Elektrokauterisation zu hemmen.

8. Vorrichtung nach Anspruch 7, wobei das Antiembolusmerkmal (340, 440) einen Stopfen (340, 440) umfasst, der in dem Lumen (326C, 426C) der Nadelkanüle (326, 426) angeordnet ist, wobei der Stopfen (340, 440) innerhalb des Lumens (326C, 426C) zwischen einer ersten Position (440A), die nahe dem distalen Ende (326B, 426B) der Nadelkanüle (326, 426) angeordnet ist, und einer zweiten Position (440B), die proximal von der ersten Position (440A) beabstandet ist, beweglich ist, wobei der Stopfen (340, 440) in der ersten Position (440A) im Wesentlichen eine Öffnung am distalen Ende (326B, 426B) der Nadelkanüle (326, 426) verschließt, um die Bildung eines Embolus innerhalb des Lumens (326C, 426C) der Nadelkanüle (326, 426) während der Elektrokauterisation zu hemmen.

9. Vorrichtung nach Anspruch 7 oder Anspruch 8, wobei das Antiembolusmerkmal (340, 440) eine kontinuierliche Druckquelle umfasst, die fluidisch mit der Nadelkanüle (326, 426) gekoppelt ist, um die Bildung eines Embolus innerhalb des Lumens (326C, 426C) der Nadelkanüle (326, 426) oder das Austreten eines Embolus aus dem Lumen (326C, 426C) der Nadelkanüle (326, 426) während der Elektrokauterisation zu hemmen.

10. Vorrichtung nach Anspruch 9, wobei das Antiembolusmerkmal (340, 440) eine kontinuierliche positive Druckquelle umfasst, die fluidisch mit der Nadelkanüle (326, 426) gekoppelt ist, um die Bildung während der Elektrokauterisation eines Embolus innerhalb des Lumens (326C, 426C) der Nadelkanüle (326, 426) zu hemmen.

11. Vorrichtung nach Anspruch 9 oder Anspruch 10, wobei das Antiembolusmerkmal (340, 440) eine kontinuierliche negative Druckquelle umfasst, die fluidisch mit der Nadelkanüle (326, 426) gekoppelt ist, um das Austreten eines Embolus aus dem Lumen (326C, 426C) der Nadelkanüle (326, 426) zu hemmen.

## Revendications

1. Appareil (20, 120, 220, 420) servant à recevoir de manière sélective un dispositif d'électrocautérisation, l'appareil comportant :
une canule d'aiguille (26, 126, 226, 326, 426) comprenant une extrémité proximale (26A) et une extrémité distale (26B, 326B, 426B), la canule d'aiguille (26, 126, 226, 326, 426) comprenant une lumière (26C, 126C, 226C, 326C, 426C) s'étendant depuis l'extrémité proximale (26A) jusqu'à l'extrémité distale (26B, 326B, 426B) ;
un manche (22, 122, 222, 422) disposé au niveau de l'extrémité proximale (26A) de la canule d'aiguille (26, 126, 226, 326, 426) ;
**caractérisé par** un récepteur d'électrocautérisation (24, 124, 224, 424) associé au manche (22, 122, 222, 422) et accouplé électriquement à la canule d'aiguille (26, 126, 226, 326, 426), ledit récepteur d'électrocautérisation (24, 124, 224, 424) comportant :
un port (24) dans le manche (22), le port (24) étant configuré pour recevoir une partie (34) du dispositif d'électrocautérisation (32) pour permettre au dispositif d'électrocautérisation (32) d'entrer directement en contact avec une partie de l'extrémité proximale (26A) de la canule d'aiguille (26) ; ou
une surface conductrice annulaire (224) accouplée électriquement à la canule d'aiguille (226), la surface conductrice (224) étant configurée pour être contactée par le dispositif d'électrocautérisation (232) ; ou
un contenant de réception (124) espacé par rapport au manche (122) et configuré pour recevoir une partie (34) du dispositif d'électrocautérisation (32), le contenant de réception étant accouplé électriquement à la canule d'aiguille (126) au moyen d'un fil (136) et étant mobile par rapport au manche ;
de telle sorte que de l'énergie électrique peut être acheminée en provenance du dispositif d'électrocautérisation (32, 132, 232) jusqu'à l'extrémité distale (26B, 326B, 426B) de la canule d'aiguille (26, 126, 226, 326, 426) à des fins d'électrocautérisation sélective du tissu en contact avec l'extrémité distale (26B, 326B, 426B) de la canule d'aiguille (26, 126, 226, 326, 426).

2. Appareil selon la revendication 1, dans lequel le récepteur d'électrocautérisation (24) comprend un port (24) dans le manche (22), le port (24) étant configuré pour recevoir une partie (34) du dispositif d'électrocautérisation (32) pour permettre au dispositif d'électrocautérisation (32) d'entrer directement en contact avec une partie de l'extrémité proximale (26A) de la canule d'aiguille (26).

3. Appareil selon la revendication 2, dans lequel le récepteur d'électrocautérisation comprend au moins deux ports (24) dans le manche (22).

4. Appareil selon la revendication 3, dans lequel chacun des ports (24) est situé au niveau d'un emplacement angulaire dans le manche (22) qui est différent de l'emplacement angulaire de chacun desdits autres un ou plusieurs ports (24).

5. Appareil selon la revendication 3 ou la revendication 4, dans lequel au moins deux desdits au moins deux ports (24) sont diamétralement opposés l'un par rapport à l'autre.

6. Appareil selon la revendication 1, dans lequel la surface conductrice comprend un anneau disposé autour du manche (222).

7. Appareil selon l'une quelconque des revendications 1 à 6, dans lequel la canule d'aiguille (326, 426) est conductrice d'énergie électrique de radiofréquence, et l'appareil comprend un dispositif anti-embole (340, 440) configuré pour inhiber toute formation, au cours d'une électrocautérisation, d'un embole à l'intérieur de la lumière (326C, 426C) de la canule d'aiguille (326, 426), ou lors de la sortie d'un embole en provenance de celle-ci.

8. Appareil selon la revendication 7, dans lequel le dispositif anti-embole (340, 440) comprend un bouchon (340, 440) disposé à l'intérieur de la lumière (326C, 426C) de la canule d'aiguille (326, 426), le bouchon (340, 440) étant mobile à l'intérieur de la lumière (326C, 426C) entre une première position (440A) se trouvant à proximité de l'extrémité distale (326B, 426B) de la canule d'aiguille (326, 426) et une deuxième position (440B) espacée de manière proximale par rapport à la première position (440A), le bouchon (340, 440) dans la première position (440A) effectuant sensiblement l'occlusion d'une ouverture au niveau de l'extrémité distale (326B, 426B) de la canule d'aiguille (326, 426) pour inhiber toute formation, au cours d'une électrocautérisation, d'un embole à l'intérieur de la lumière (326C, 426C) de la canule d'aiguille (326, 426) .

9. Appareil selon la revendication 7 ou la revendication 8, dans lequel le dispositif anti-embole (340, 440) comprend une source de pression continue accouplée de manière fluidique à la canule d'aiguille (326, 426) pour inhiber toute formation, au cours d'une électrocautérisation, d'un embole à l'intérieur de la lumière (326C, 426C) de la canule d'aiguille (326, 426), ou lors de la sortie d'un embole en provenance de l'intérieur de celle-ci.

10. Appareil selon la revendication 9, dans lequel le dispositif anti-embole (340, 440) comprend une source de pression positive continue accouplée de manière fluidique à la canule d'aiguille (326, 426) pour inhiber toute formation, au cours d'une électrocautérisation, d'un embole à l'intérieur de la lumière (326C, 426C) de la canule d'aiguille (326, 426).

11. Appareil selon la revendication 9 ou la revendication 10, dans lequel le dispositif anti-embole (340, 440) comprend une source de pression négative continue accouplée de manière fluidique à la canule d'aiguille (326, 426) pour inhiber toute sortie d'un embole en provenance de l'intérieur de la lumière (326C, 426C) de la canule d'aiguille (326, 426).
